# EUROPEAN PATENT SPECIFICATION

(11) **EP 4 499 185 B1**
(45) Date of publication and mention of the grant of the patent: **11.11.2026**
(21) Application number: 23707725.0
(22) Date of filing: 27.02.2023
(51) Int. Cl.: A61M 11/00, A61M 15/00, A61M 15/08

(54) **FLUID DELIVERY DEVICE**
FLÜSSIGKEITSABGABEVORRICHTUNG
DISPOSITIF DE DISTRIBUTION DE FLUIDE

(30) Priority: 31.03.2022 GB 202204727
(43) Date of publication of application: 05.02.2025
(60) Divisional application: 26208339.7 / 4 837 604
(73) Proprietor: Bespak Europe Limited, North Lynn Industrial Estate King's Lynn Norfolk PE30 2JJ (GB)
(72) Inventor: GIBBONS, Adam, Norfolk PE30 2JJ (GB)
(74) Representative: Boult Wade Tennant LLP
(86) International application number: PCT/EP2023/054806
(87) International publication number: WO 2023/186422

(56) References cited:
- EP-A1- 0 546 607
- WO-A1-2015/008048
- US-A1- 2012 234 861
- US-A1- 2014 231 457
- US-A1- 2022 080 134

## Description

The present disclosure relates to a fluid delivery device. In particular, it relates to a fluid delivery device for nasal, sub-lingual or ophthalmic use.

### Background

Fluid delivery devices are known for dispensing media such as powders and fluids for discharge into or onto the body.

WO2015008048 discloses a fluid delivery device for discharging a fluid comprising: a housing; piston pump; biasing mechanism; and trigger mechanism. The housing comprises a basal end and a discharge end having an outlet for discharging the fluid. The piston pump comprises a casing defining a pump chamber for storage of the fluid, piston slidably movable relative to the pump chamber, piston plunger, and delivery channel. The trigger mechanism is movable from a cocked configuration wherein the trigger mechanism prevents transfer of a biasing force of the biasing mechanism to the casing to thereby prevent movement of the casing towards the outlet of the housing to a triggered configuration wherein the trigger mechanism enables transfer of the biasing force to the casing to thereby cause movement of the casing towards the outlet of the housing so as to discharge fluid from the pump chamber, along the delivery channel and out of the outlet of the housing. The biasing mechanism comprises one or more flexible catch members and a manually operable trigger.

US2012234861 discloses a fluid delivery device for discharging a fluid having a housing, a piston pump, a biasing mechanism, and a trigger mechanism. The housing has a fluid discharge outlet. The pump has a pump chamber casing for fluid storage, a piston sliding in the pump chamber, a piston plunger, and a delivery channel leading to the housing outlet The biasing mechanism biases the piston pump casing towards the housing outlet. The trigger mechanism has a catch member and a restraining member movable by a manually actuatable trigger from a cocked to a triggered configuration. The cocked restraining member engages the catch member to prevent movement and fluid discharge. A triggered restraining member disengages from the catch member for movement of the casing towards the housing outlet to discharge fluid from housing outlet.

US2014231457 A1 discloses a fluid delivery device for discharging a fluid disclosing trigger mechanism with a slidable button and a first and second movement phase.

The closure of assembly gaps and/or clearance spaces between the components of a mechanism on triggering of a biased mechanism may result in noise on actuation of a device.

Unwanted movement of the container before triggering a biased mechanism may result in unwanted dispensing of fluid. For example, this may occur if the fluid delivery device were to be dropped in an orientation which would tend urge the container towards the outlet. In such a movement the container may move towards a piercer or relative to a plunger without the trigger mechanism having been moved, resulting in loss of the fluid from the vial before actuation of the mechanism.

### Summary of the Disclosure

According to the present invention there is provided a fluid delivery device according to claim 1.

Optionally, the engagement element comprises one or more flexible arms. The or each of the flexible arms may be configured to be inserted into an open end of the container during movement of the container towards the outlet of the housing and may be optionally biased outwards to thereby engage with an inner face and/or upper edge of the container. The or each of the flexible arms may be biased towards a centre of the container to thereby create frictional engagement with the container, wherein the or each of the flexible arms may optionally be in frictional engagement with an outer face of the container. The or each of the flexible arms may extend along or in the direction of a longitudinal axis of the device; and/or the or each of the flexible arms may extend circumferentially around the container when the container is in the storage configuration.

The or each of the flexible arms may be arranged between the container and the discharge end of the housing when the container is in the storage configuration. The or each of the flexible arms may be arranged laterally to the container and extend along at least part of a length of the container when the container is in the storage configuration. The or each of the flexible arms may extend from an extension of the housing.

The engagement element may comprise one or more protrusions configured to contact a surface of the container to thereby exert a force on the container to selectively resist movement of the container towards the outlet of the housing. Each of the one or more flexible arms may comprise one of the one or more protrusions. The or each of the one or more protrusions may comprise one or more elongate ribs arranged around a circumference of the container.

The engagement element may be configured to engage the container to thereby selectively resist movement of the container towards the outlet of the housing. The engagement element may be configured to engage one or more of an outer face, an inner face or a rim of an open end of the container.

The container may comprise at least one recess and/or at least one protrusion configured to engage with the engagement element.

The fluid delivery device may further comprise a container holder configured to hold the container, wherein the engagement element is arranged on the container holder.

The trigger mechanism comprises a slidable button, the slidable button being configured to push the container towards the outlet of the housing.

The trigger mechanism may comprise one or more flexible catch members that in the cocked configuration are catched to the housing and in the triggered configuration are decoupled from the housing; and the slidable button may comprise an obstructer that in the cocked configuration engages the one or more flexible catch members to prevent decoupling of the one or more flexible catch members from the housing.

The trigger mechanism may comprise a slide that is biased by action of the biasing member, the slide being arranged to apply the biasing force to the slidable button in the triggered configuration; and the slide may comprise a container holder configured to hold the container, wherein the engagement element is arranged on or integrated with the container holder.

The fluid delivery device may further comprise:
a piercer defining a delivery channel for delivering fluid towards the outlet of the housing; and
a pierceable stopper arranged within the chamber, the pierceable stopper being configured to be pierced by the piercer so as to discharge the fluid from the chamber along the delivery channel and out of the outlet of the housing.

The piercer may comprise a needle and a needle holder, wherein the engagement element is arranged on the needle holder, the needle holder optionally being fixed in position relative to the housing.

The device may be a single-use device.

There is also provided a fluid delivery device as described above combined with a single dose of fluid contained in the chamber of the container. The fluid may contain a pharmaceutical. The term pharmaceutical, as used herein, is intended to encompass any pharmaceutical, compound, composition, medicament, agent or product which can be delivered or administered to a human being or animal, for example pharmaceuticals, drugs, biological and medicinal products. Examples include antiallergics, analgesics, bronchodilators, antihistamines, therapeutic proteins and peptides, antitussives, anginal preparations, antibiotics, anti-inflammatory preparations, hormones, CNS treatments targeting the blood brain barrier, or sulfonamides, such as, for example, a vasoconstrictive amine, an enzyme, an alkaloid, or a steroid, including combinations of two or more thereof. In particular, examples include isoproterenol [alpha-(isopropylaminomethyl) protocatechuyl alcohol], phenylephrine, phenylpropanolamine, glucagon, adrenochrome, trypsin, epinephrine, ephedrine, narcotine, codeine, atropine, heparin, morphine, dihydromorphinone, ergotamine, scopolamine, methapyrilene, cyanocobalamin, terbutaline, rimiterol, salbutamol, ipratropium bromide and salbutamol, flunisolide, colchicine, pirbuterol, beclomethasone, orciprenaline, fentanyl, diamorphine, Midazolam, vagezpant, epinephrine, ketamine, zolmitriptan, dexmedetomidine, naloxone, Nalmefene, streptomycin, penicillin, procaine penicillin, tetracycline, chlorotetracycline and hydroxytetracycline, adrenocorticotropic hormone and adrenocortical hormones, such as cortisone, hydrocortisone, hydrocortisone acetate and prednisolone, insulin, cromolyn sodium, and mometasone, including combinations of two or more thereof.

The pharmaceutical may be used as either the free base or as one or more salts conventional in the art, such as, for example, acetate, benzenesulphonate, benzoate, bicarbonate, bitartrate, bromide, calcium edetate, camsylate, carbonate, chloride, citrate, dihydrochloride, edetate, edisylate, estolate, esylate, fumarate, fluceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylbromide, methylnitrate, methylsulphate, mucate, napsylate, nitrate, pamoate, (embonate), pantothenate, phosphate, diphosphate, polygalacturonate, salicylate, stearate, subacetate, succinate, sulphate, tannate, tartrate, and triethiodide, including combinations of two or more thereof. Cationic salts may also be used, for example the alkali metals, e.g. Na and K, and ammonium salts and salts of amines known in the art to be pharmaceutically acceptable, for example glycine, ethylene diamine, choline, diethanolamine, triethanolamine, octadecylamine, diethylamine, triethylamine, 1-amino-2-propanol-amino-2-(hydroxymethyl)propane-1,3-diol, and 1-(3,4-dihydroxyphenyl)-2 isopropylaminoethanol.

The pharmaceutical will typically be one which is suitable for nasal inhalation and may be provided in any suitable fluid form for this purpose, for example as a solution or powder suspension in a solvent or carrier liquid. The solution may be an aqueous solution or use another solvent, for example ethanol, or isopropyl alcohol. The pharmaceutical may alternatively be one suitable for sub-lingual or ophthalmic delivery.

The pharmaceutical may, for example, be one which is suitable for the treatment of asthma. Examples include salbutamol, beclomethasone, salmeterol, fluticasone, formoterol, terbutaline, sodium chromoglycate, budesonide and flunisolide, and physiologically acceptable salts (for example salbutamol sulphate, salmeterol xinafoate, fluticasone propionate, beclomethasone dipropionate, and terbutaline sulphate), solvates and esters, including combinations of two or more thereof. Individual isomers such as, for example, R-salbutamol, may also be used. The pharmaceutical may, for example, be one which is suitable for the treatment of migraine. An example is sumatriptan. As will be appreciated, the pharmaceutical may comprise of one or more active ingredients, an example of which is flutiform. One or more surfactants may be included if desired.

The pierceable stopper may be formed from an elastomer or thermoelastomer material such as EPDM, polychloroprene, hydrogenated nitrile, butyl, halo-butyl, dynamically crosslinked EPDM/PP (Santoprene^{®}), styrenic block copolymers or blends thereof. Other suitable materials include high-density polyethylene and low-density polyethylene. The housing may be formed from an engineering plastic such as polypropylene, HDPE, ABS, polyester or POM.

The container may be formed from any suitable material such as glass, cyclic olefin copolymer - an example being Topas^{®} COC available from TOPAS Advanced Polymers GmbH, Frankfurt-Höchst, Germany, or liquid crystal polymer - an example being Zenite^{®} LCP available from DuPont, Wilmington, USA.

Components of the biasing mechanism, trigger mechanism and housing may be formed from a suitable material having the necessary flexural characteristics, such as polypropylene, HDPE, ABS, polyester, POM, PBT or polypropylene.

By providing a moveable container, any spaces between components which are present after assembly can be taken up before the container moves to the dispensing configuration, thereby reducing noise during dispensing. By providing the engagement elements, forces in the device may be balanced to limit the possibility of unintentional or early triggering of the trigger mechanism, and the risk of unintended movement of the moveable container may be reduced. The engaging element may balance forces in the trigger mechanism such that the risk of early triggering is reduced or eliminated.

By arranging the slidable button to push the container towards the outlet of the housing before the triggering mechanism is triggered, actuation noise may be reduced and/or potential damage from spring recoil may be avoided.

The risk of accidental dispensing of fluid may be reduced by use of the engagement element configured to selectively resist movement of the container towards the outlet of the housing, the resistance of the engagement element being overcome during movement of the slidable button but before the trigger mechanism is moved to the triggered configuration,. Further, such a mechanism allows for improved control of the release force required to push the slidable button to the triggered configuration.

By providing an engagement element which is bypassed before the trigger mechanism is moved to the triggered configuration, the possibility of the additional resistance of the engagement element hindering the output of the biasing element during actuation may be avoided.

By providing an engagement element in which the or each of the flexible arms is in a neutral, unbiased position in the cocked configuration, the device may be more robust due to avoiding the influence of material creep and/or manufacturing tolerances.

### Brief description of the drawings

The present disclosure will now be described, by way of example only, with reference to the accompanying drawings in which:
Figure 1 is a schematic cross-section view of a fluid delivery device according to a first example with the container in the storage configuration and the trigger mechanism in the cocked configuration;
Figure 2 is a schematic cross-section view of the device of Figure 1 with the container in the storage configuration and the trigger mechanism in the triggered configuration;
Figure 3 is a schematic cross-section view of the device of Figure 1 with the container in the dispensing configuration and the trigger mechanism in the triggered configuration;
Figure 4 is a schematic cross-section view of a fluid delivery device according to a second example with the container in the storage configuration and the trigger mechanism in the cocked configuration;
Figure 5 is a schematic partial plan view on X-X of the container and container holder of the device of Figure 4, omitting the other components for clarity;
Figure 6 is a schematic cross-section view of a fluid delivery device according to a third example with the container in the storage configuration and the trigger mechanism in the cocked configuration, with the structure of the housing around the button omitted for clarity;
Figure 7 is a schematic cross-section view of the device of Figure 6 with the container in the storage configuration and the trigger mechanism in the triggered configuration;
Figure 8 is a schematic cross-section view of a fluid delivery device according to a fourth example with the container in the storage configuration and the trigger mechanism in the cocked configuration; and
Figure 9 is a schematic partial plan view of the container and container holder of the device of Figure 8, omitting the other components for clarity.

### Description

In the following, the fluid delivery device will be described, by way of example only, as a device suitable for nasal delivery of fluid.

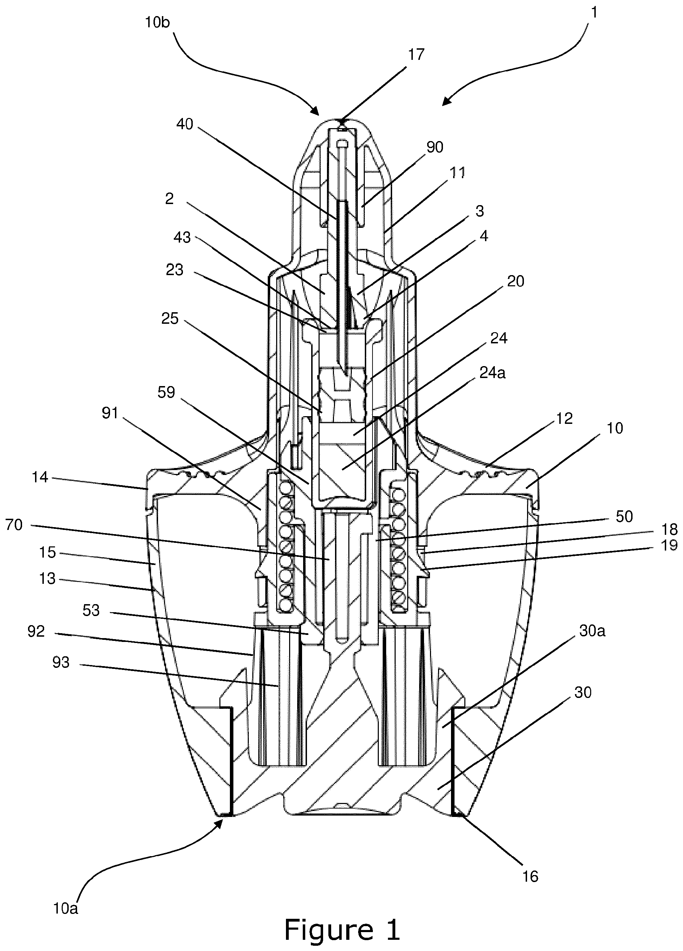

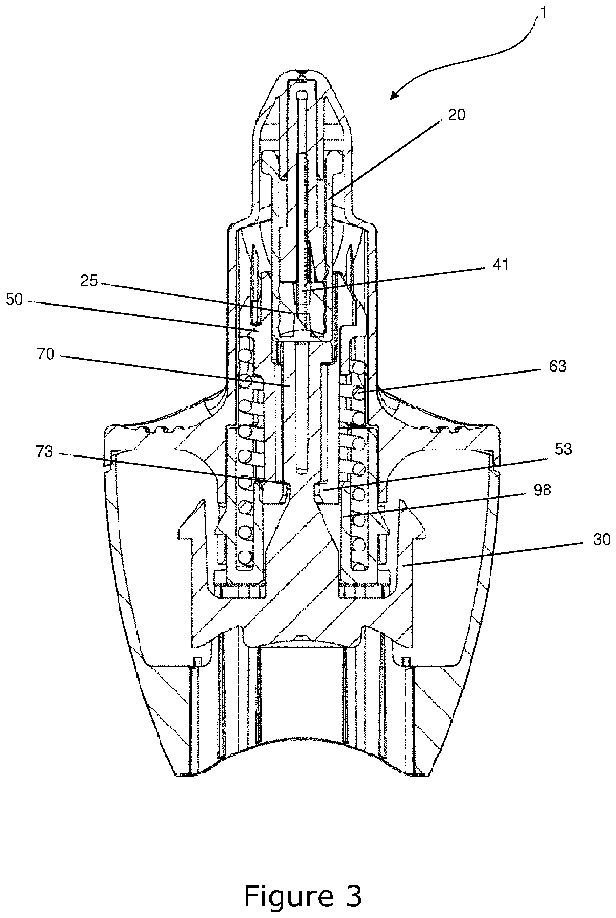

In a first example as shown in Figures 1 to 3, a fluid delivery device 1 for discharging a fluid comprises a housing 10 having a basal end 10a and a discharge end 10b, the discharge end 10b having an outlet for discharging the fluid. The fluid delivery device 1 comprises a container 20 defining a chamber for storage of the fluid, the container 20 being slideably moveable relative to the housing 10 from a storage configuration (as shown in Figure 1) to a dispensing configuration (as shown in Figure 3). The fluid delivery device 1 also comprises an engagement element 2 configured to selectively resist movement of the container 20 towards the outlet of the housing 10.

The housing 10 of the fluid delivery device 1 may be formed from two parts, an upper housing 14 and a lower housing 15. The upper housing 14 and the lower housing 15 may be connected by means of co-operating formations 18 and 19. A lower rim of the upper housing 14 and an upper rim of the lower housing 15 may interface with one another on assembly of the device 1. An optional additional snap-fit arrangement or ultrasonic weld may be provided at this interface if desired.

The housing 10 may define an interior for containing the remaining components of the device 1. The device 1 may be assembled by connecting together the upper housing 14 and the lower housing 15 around the other components. In particular the co-operating formations 18 and 19 of the upper housing 14 and the lower housing 15 may snap-fit together and retain the upper housing 14 and the lower housing 15 together so as to be resistant to easy disassembly of the device 1.

The upper housing 14 and the lower housing 15 may together define a side wall 13 of the housing 10, a base 16 defining the basal end and opposite the base 16 a nasal piece 11 which extends upwardly towards an outlet in the form of an orifice 17 which is provided at the discharge end. A longitudinal axis of the device 1 may run from the base 16 to the orifice 17. Shoulder portions 12 may be provided between the nasal piece 11 and the side walls 13.

The nasal piece 11 may be provided with a first extension 90, which may be tubular, which may extend downwardly from an upper end of the nasal piece 11 within the interior of the device 1. A swirl chamber formation 11a may be provided at an upper end of the nasal piece 11 adjacent the orifice 17.

The upper housing 14 may be provided with a second extension 91, which may also be tubular, that may extend downwardly from the shoulder portions 12 towards the base 16. Towards a lower end of the second extension 91 may be located two apertures that form the co-operating formation 18 of the upper housing 14 on opposite sides of the second extension 91. The bottom end of the second extension 91 is open.

The lower housing 15 may comprise the base 16. The base 16 may comprise a recess 93 formed by a third extension 92 formed as part of the lower housing 15. The third extension 92 may be tubular and extend upwardly from the base 16.

The third extension 92 may comprise a lower portion 94 and an upper portion 95 that may be inter-connected by a plurality of webs (not visible in the Figures) with apertures being defined in between the webs. The upper portion 95 may have a smaller diameter than the lower portion 94.

As most clearly shown in Figure 1, the upper portion 95 may comprise an inner extension 98, that may be tubular, creating an annular chamber 97. The inner extension 98 may extend upwardly from a lower flange 99 that closes of the lower end of the annular chamber 97. The upper portion 95 may be provided with outwardly extending projections that form the co-operating formation 19 of the lower housing 15.

The lower housing 15 including the lower portion 94 and upper portion 95 of the third extension 92 may be formed as a single unitary part, for example as a moulded part.

A piercer 40 may be provided within the housing 10. The piercer 40 may be coupled to the first extension 90 and extend downwardly therefrom. The piercer 40 may comprise a needle 41 and a needle holder 42. The needle 41 and needle holder 42 may form a subassembly. The piercer 40 may be inserted into and retained by the first extension 90. The needle 41 may define at least a portion of a delivery channel that extends to the outlet at orifice 17. The needle 41 may be fixed relative to the upper housing 14.

As shown in Figures 1 to 3, the container 20 may comprise a generally cylindrical body 21 which has a closed base 22 at one end and an upper opening 23 at an end opposite the closed base 22. The container 20 defines a chamber 24 in which in use a fluid 24a may be stored prior to discharge on operation of the device 1. The generally cylindrical body 21 may comprise a thickened portion at its upper end. This thickened portion may form an external protruding ridge 29 extending circumferentially around the generally cylindrical body 21.

The pierceable stopper 25 may be slideably moveable within the chamber 24.

The pierceable stopper 25 may comprise a central portion 26 configured to be pierced by the piercer 40 and a sealing portion 27 arranged around the central portion 26 for sealing to an internal surface of the container 20. The central portion 26 may be relatively thinner than the sealing portion 27 in an axial direction of the container 20.

The pierceable stopper 25, and in particular the sealing portion 27, may comprise one or more sealing rings 28 on an exterior surface that provide for a sliding seal interface with an internal surface of the cylindrical body 21 of the container 20.

The fluid delivery device 1 comprises a trigger mechanism configured to apply a force to the container 20 to move the container 20 towards the outlet of the housing 10. The trigger mechanism may thereby move the container 20 towards the dispensing configuration.

The trigger mechanism may be configured such that movement occurs in two phases. In a first movement phase the trigger mechanism may push the container 20 from the storage configuration (as shown in Figure 1) towards the outlet of the housing 10 to overcome the resistance of the engagement element 2. The first movement phase may end once the resistance of the engagement element 2 is overcome. For example, the container 20 may be moved far enough such that the engagement element 2 is overcome by being physically bypassed or deflected before the container 20 reaches the dispensing configuration. The device 1 may be configured such that the engagement element 2 is overcome before the container 20 reaches the dispensing configuration.

The trigger mechanism comprises a manually operated slidable button 30 located at the basal end of the housing 10. The slidable button 30 is configured to push the container 20 towards the outlet of the housing 10 during the first movement phase to overcome the resistance of engagement element 2. The slidable button 30 may be formed as a rigid unitary component.

The trigger mechanism may further be configured to push the container 20 further towards the outlet of the housing 10 in a second movement phase the such that the container 20 is moved to the dispensing configuration (as shown in Figure 3).

The fluid delivery device 1 may comprise a biasing element 63, shown in the example of Figures 1 to 3 in the form of a spring. In the first movement phase, the trigger mechanism may be arranged in a cocked configuration (as shown in Figure 1) in which the trigger mechanism is configured to prevent transfer of a biasing force of the biasing element 63 to the container 20, such that the container 20 is moved under the action of the manual operation of the slidable button 30. The first movement phase, including overcoming the resistance of engagement element 2, may occur while the trigger mechanism is in the cocked configuration.

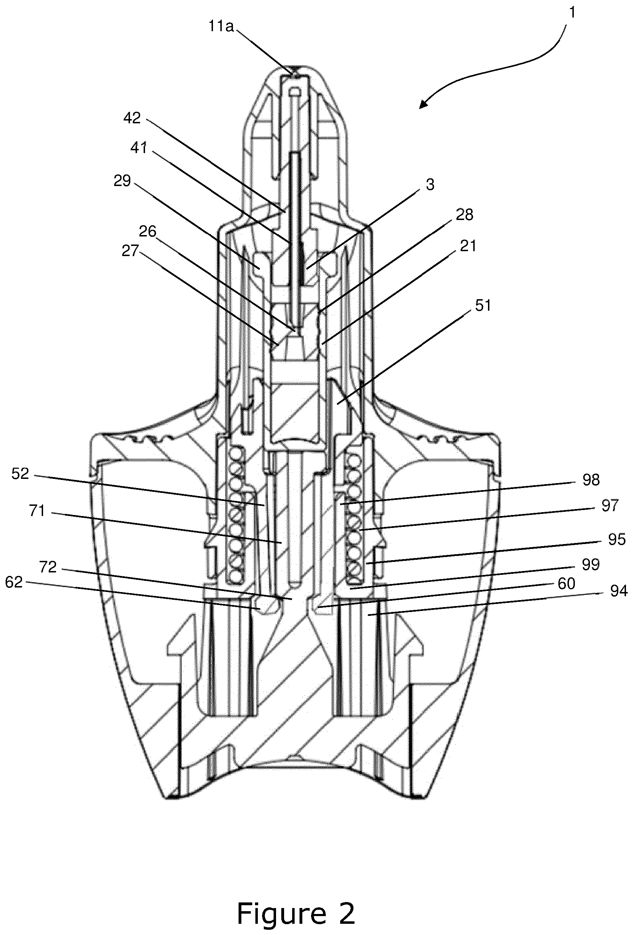

The trigger mechanism is be configured to move to a triggered configuration during the second movement phase. The trigger mechanism is moved to the triggered configuration by continued manual actuation of the slidable button 30. In the triggered configuration, the trigger mechanism may apply the biasing force of the biasing element 63 to the container 20 to thereby push the container 20 towards the outlet of the housing 10 such that the container 20 is moved to the dispensing configuration. Figure 2 shows the device of Figure 1 after the trigger mechanism has moved to the triggered configuration, at the point when the biasing element 63 is free to move the container 20 to the dispensing configuration because the trigger mechanism is not catched to the housing 10. In examples in which the biasing element 63 is a spring, the spring may be compressed when the trigger is in the cocked configuration, and the force may be transferred by extension of the spring towards its resting position when the trigger mechanism is in the triggered configuration.

The trigger mechanism may comprise a slide 50 that is biased by action of the biasing element 63. The slide 50 may comprise an upper end 51 and a plurality of dependent legs 52 that may extend downwardly from the upper end 51. Three, four or more dependent legs 52 may be provided. A lower end of the biasing element 63 may be seated within the annular chamber 97 of the third extension 92.

The upper end 51 may comprise a cup-shaped extension 59 that is shaped and sized to engage a lower portion of the container 20. The cup-shaped extension 59 may contact the closed base 22 and/or a lower end of the generally cylindrical body 21. The cup-shaped extension 59 may therefore act as a container holder for holding container 20 when the device is in the storage configuration.

The trigger mechanism may comprise one or more flexible catch members that in the cocked configuration are catched to the housing 10 (as shown in Figure 1) and in the triggered configuration are decoupled from the housing (as shown in Figure 3). The legs 52 may each comprise a flexible catch member in the form of an enlarged catch portion 53 arranged at or near a lower end of the leg 52. Each enlarged catch portion 53 may comprise an inwardly directed projection 60 and an outwardly directed projection 62. An outer edge of the enlarged catch portion 53 may be provided with a downward facing chamfer. An inner edge of a lower face of the projection 60 may be provided with a downward facing chamfer. An outer edge of an upper face of the projection 62 may be provided with an upward facing chamfer.

A lower face of the slidable button 30 may form a trigger surface that can be contacted in use by a finger or thumb of a user.

The slidable button 30 may comprise a cup-shaped body 30a comprising, or coupled to, an inwardly directed projection that comprises an obstructer 70. When the trigger mechanism is in the cocked configuration, the obstructer 70 may engage the one or more flexible catch members to prevent decoupling of the one or more flexible catch members from the housing 10. As shown most clearly in Figures 1 to 3, the obstructer 70 may comprise an upper, enlarged section 71 and a lower, waisted section 72. A shoulder 73 may be formed at the junction between the enlarged section 71 and the waisted section 72.

The enlarged section 71 may extend up and into the vicinity of the bottom of the container 20. A small assembly gap may be provided therebetween if desired. In the Figures the enlarged section 71 is shown as hollow. A solid enlarged section 71 may be used as an alternative.

The engagement element 2 may comprise one or more flexible arms 3. In the illustrated example the engagement element 2 comprises multiple flexible arms 3. The one or more flexible arms 3 may extend from the needle holder 42 in a direction generally aligned with the longitudinal axis of the device and may be arranged around a circumference of the needle holder 42. The flexible arms 34 may therefore extend from the first extension 90 towards the container 20 such that they are arranged between the container 20 and the discharge end of the housing 10 when the container 20 is in the storage configuration. A distal end of the or each flexible arm 3 may extend into an open end of the container 20 in the storage configuration. As the container 20 is moved from the storage configuration as described in more detailed below, the or each flexible arm 3 may be inserted further into the container 20.

The or each of the flexible arms 3 may comprise a projection 4 arranged on an outer face of the flexible arm 3. The projection 4 may be arranged at or near a the distal end of the flexible arm 3 such that the projection 4 engages an inner face, upper rim or projection of the container 20 as the container 20 is moved from the storage configuration. Therefore, the flexible arm 3 and/or projection 4 may selectively resist movement of the container 20 towards the outlet of the housing 10.

The flexible arms 3 and/or the projections 4 may flex inwards as the container 20 contacts the engagement element 2 such that the container 20 can move past a point at which flexible arms 3 and/or the projections 4 resist movement of the container 20, thereby overcoming the resistance of the engagement element. The flexible arms 3 and/or the projections 4 may be biased outwards to improve engagement with an inner face and/or upper edge of the container 20. Alternatively, the flexible arms 3 and/or the projections 4 may be arranged in a neutral, unbiased position when the device is in the storage configuration.

The arrangement of the flexible arms 3 and/or the position of the projections 4 on the flexible arms relative to the container 20 may be configured such that the container 20 moves past the point at which the flexible arms 3 and/or the projections provide resistance before the trigger mechanism is moved to the triggered configuration.

The one or more flexible arms may further comprise an end face 43 for abutting the pierceable stopper 25 in use. The end face 43 may be axially offset from a tip of the needle 41 by a fixed length such that the end face 43 abuts the pierceable stopper 25 when the needle 41 has pierced through the pierceable stopper 25 by a fixed length. The end face 43 may be configured to push the pierceable stopper 25 further into the container 20 to expel the fluid from the chamber 24 on further movement of the container 20.

In use, the device 1 is actuated to dispense the fluid by moving the container 20 from the storage configuration to the dispensing configuration. This movement may be achieved by manually pushing up on the trigger surface of slidable button 30, causing the obstructer 70 to move upwards.

Figure 1 shows the fluid delivery device 1 with the container 20 in the storage configuration and the trigger mechanism in the cocked configuration. In the cocked configuration the enlarged catch portions 53 of the legs 52 may be engaged against the housing 10, and in particular against the inner extension 98 and or the lower flange 99. The outwardly directed projection 62 may be catched under the lower flange 99. The upward facing chamfer of the outwardly directed projection may abut a chamfer provided at the intersection of the inner extension 98 and the lower flange 99. Catching of the outwardly directed projection 62 may prevent upward movement of the legs 52 and transfer of the biasing force of biasing element 63 onto the container 20. At the same time inward deflection of the legs 52 to uncatch the outwardly directed projection 62 may be prevented by the presence of the obstructer 70 since the inwardly directed projection 60 is butted against the exterior face of the obstructer 70, and in particular the enlarged section 71. The inwardly directed projection 60 may also be catched under the shoulder 73.

During actuation, in the first movement phase upward movement of the obstructer 70 may cause the distal end of the enlarged section 71 to contact the lower end of the container 20 by closing the assembly gap between the obstructer 70 and the container 20.

Further upward movement may cause the container 20 to move upwards relative to the housing 10 such that at least a portion of the container 20 moves past or otherwise overcomes the resistance of engagement element 2 before the trigger mechanism is moved to the triggered configuration.

After the resistance of the engagement element 2 is overcome, in the second movement phase, the obstructer 70 may continue to push the container 20 further towards the outlet of the housing 10. This further upward movement may cause the container 20 and pierceable stopper 25 (as one) to move upwards relative to the housing 10, and therefore relative to the piercer 40, such that the pierceable stopper 25 is pierced by the tip of the needle 41, preferably through the central portion 26.

As the slidable button 30 continues to be moved manually upwards, the enlarged section 71 of the obstructer 70 may be moved out of alignment with the enlarged catch portions 53. Once the waisted section 72 is aligned with the enlarged catch portions 53 the legs 52 are flexed together under the force of the biasing element 63 to engage the projection 60 under the shoulder 73.

Figure 2 shows the fluid delivery device 1 at this point, during triggering of the trigger mechanism. The slide 50 is decoupled from the lower housing 15 and as a result the slide 50 is free move rapidly upwards under the biasing force of biasing element 63. During this portion of the movement the legs 52 are constrained to remain inwardly flexed, and engaged under shoulder 73, due to the internal diameter of the inner extension 98 against which the enlarged catch portions 53 slide.

Further upward movement of the slide 50 may therefore be driven by the biasing force of the biasing element 63 and carries with it the obstructer 70 and the container 20. Thrusting force may be applied to the container 20 by a shoulder of the cup-shaped extension 59 and/or by the distal end of the enlarged section 71 of the obstructer 70 contacting the closed base 22 of the container 20.

The rapid upward movement may cause further relative movement of the pierceable stopper 25 within the container 20 (as the container 20 is thrust upwards) that pressurises the chamber 24 causing the fluid therein to be dispensed up the needle 41 and out of the orifice 17 via the swirl chamber formation 11a as an atomised spray.

Figure 3 shows the fluid delivery device 1 after dispensing of the fluid has been completed, with the obstructer 70, slide 50 and container 20 displaced fully upwards.

The point of piercing of the pierceable stopper 25 (i.e. the point at which the container is in the dispensing configuration) may be configured to occur during the manual movement of the container 20 before the device 1 achieves the triggered configuration, preferably during the second movement phase. Alternatively, the point of piercing of the pierceable stopper 25 may be configured to occur during the movement of the container 20 driven by the biasing force of the biasing element 63, i.e. after the triggered configuration has been achieved. The different configurations may be achieved by suitable sizing of the lengths of the obstructer 70 and needle 41 and location of the pierceable stopper 25.

Since any assembly gaps of the device are closed prior to the slide 50 being decoupled, the activation noise may be significantly reduced. Activation noise is also reduced by the presence of the inner extension 98 as it helps to prevent recoil and 'snaking' of the spring which may occur where a larger void space is provided surrounding the spring.

Additionally using one or more assembly gaps may beneficially separate the frictional interfaces between the components during the actuation stroke, thus reducing the peak actuation force required. For example, the static friction between the slidable button 30 and the enlarged catch portions 53 can be overcome (to cause relative movement there between) before the static friction between the container 20 and the pierceable stopper 25 and/or piercer needs to be overcome.

The device 1 may be configured such that the slide 50 moves a predetermined actuation distance on actuation of the device. The inner extension 98 may have a length equal to or greater than this actuation distance. As a result, after actuation, as shown in Figure 3, the legs 52 may be retained within the bore of the inner extension 98 with the enlarged catch portions 53 still engaged under the shoulder 73. This may prevent the slidable button 30 moving back downwards to its initial position. This is advantageous in that it acts as a visual and tactile indicator that the device has been activated. In addition it provides a stable post-actuation structure in which the components are not free to be rattled about.

While the description of the device above describes the enlarged section 71 directly contacting the container 20, it will be appreciated that the closure of the assembly gaps may also be achieved by indirect contact through an intermediate component between the projecting part and the container 20. For example, the enlarged section 71 may be formed from two or more pieces that are moved in turn.

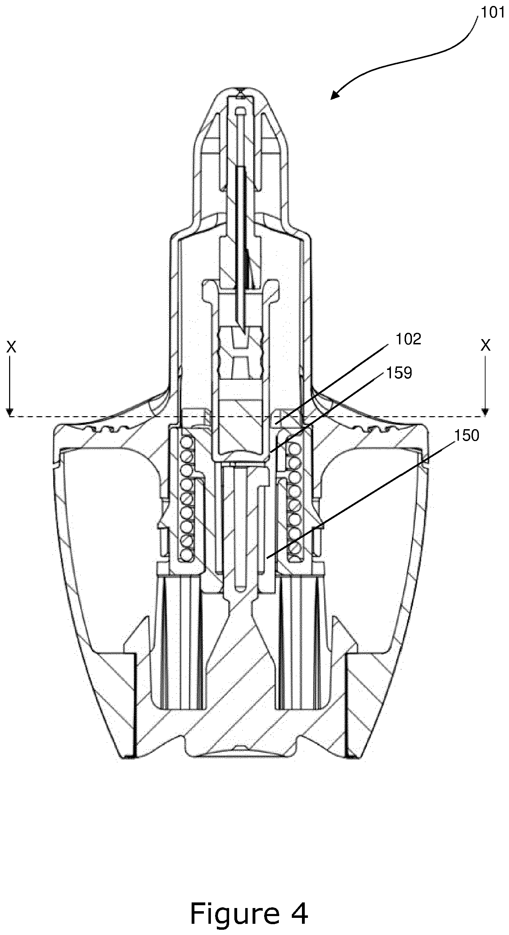

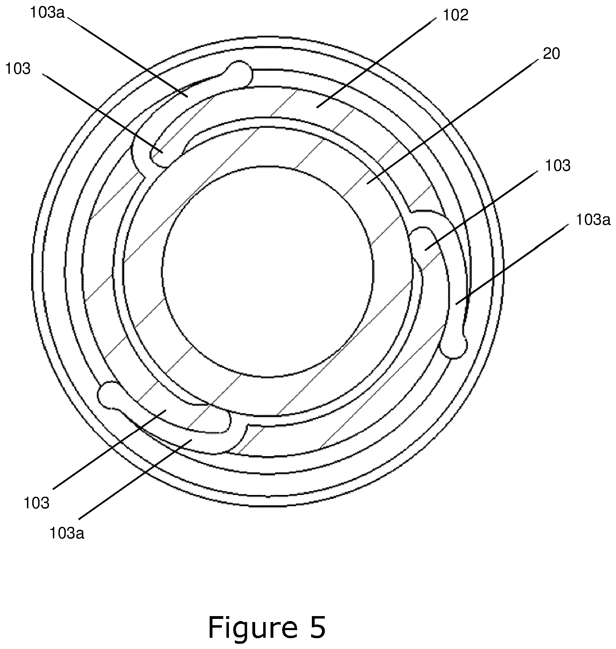

Figures 4 and 5 show another example of a fluid delivery device 101 according to the present disclosure. Only the differences compared to the fluid delivery device 1 previously described will be set out in detail. In all other respects the fluid delivery device 101 may be the same at that already described. Compared to the previous fluid delivery device 1, the fluid delivery device 101 of Figures 4 and 5 has a different configuration of the engagement element 102.

The engagement element 102 may comprise one or more flexible arms 103. In the illustrated example the engagement element 102 comprises multiple flexible arms 103. The flexible arms 103 may be arranged at an upper end of the cup-shaped extension 159 of the slide 150, the cup-shaped extension 159 forming a container holder for holding the container 20 when the container is in the storage configuration. The flexible arms 103 may be formed integrally with the cup-shaped extension 159. The flexible arms 103 may extend generally circumferentially such that they extend around the container 20 when the container 20 is in the storage configuration.

A recess or opening 103a may be provided to allow the or each of the flexible arms 103 to flex outwards from their rest position when the container 20 is received in the flexible arms 103 (for example, when the container 20 is in the storage configuration).

The flexible arms 103 may be biased towards a centre of the container to thereby create frictional engagement with the container when the container 20 is received in the flexible arms 103 (for example, when the container 20 is in the storage configuration). Therefore, the flexible arms 103 may selectively resist movement of the container 20 towards the outlet of the housing 10.

In this example, the flexible arms 103 may remain in contact with the container 20 throughout displacement of the container 20 or until the trigger mechanism moves to the triggered configuration. The resistance of the flexible arms 103 may be overcome when sufficient force is applied to overcome the friction between the flexible arms 103 and the container 20, i.e. when the container 20 is moved sufficiently relative to the engagement element that the engagement element provides reduced or no resistance to further movement of the container 20.

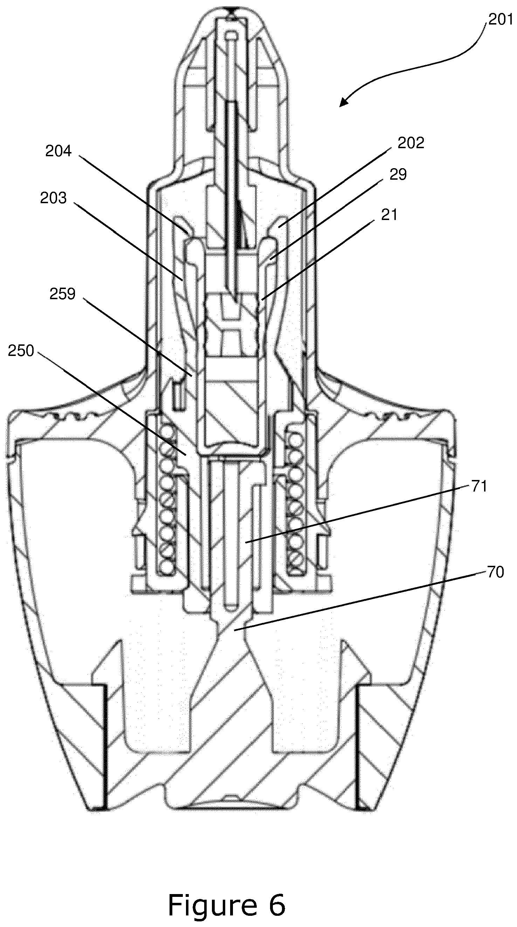

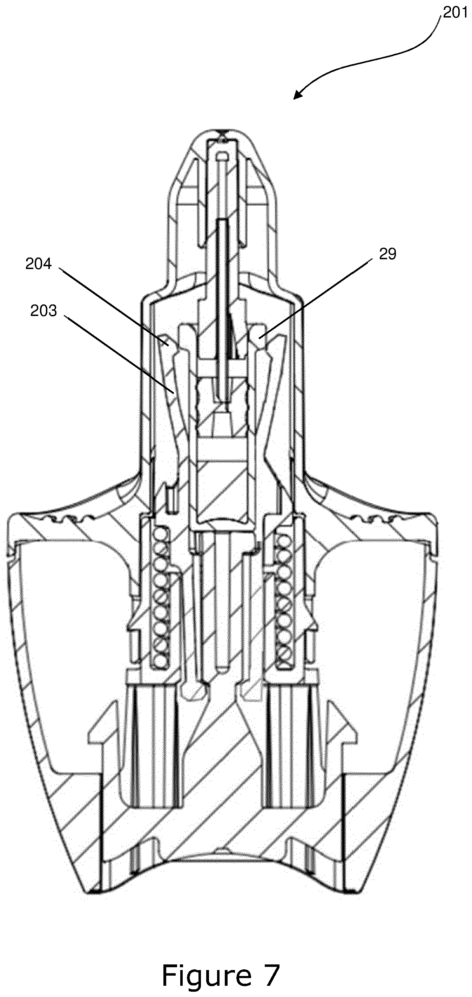

Figures 6 and 7 show another example of a fluid delivery device 201 according to the present disclosure. Only the differences compared to the fluid delivery device 1 previously described will be set out in detail. In all other respects the fluid delivery device 201 may be the same at that already described. Compared to the previous fluid delivery device 1, the fluid delivery device 201 of Figures 6 and 7 has a different configuration of the engagement element 202.

The engagement element 202 may comprise one or more flexible arms 203. In the illustrated example the engagement element 202 comprises multiple flexible arms 203. The flexible arms 203 may form part of the lateral walls of the cup-shaped extension 259 of the slide 250, the cup-shaped extension 259 forming a container holder for holding the container 20 when the container is in the storage configuration. The flexible arms 203 may be formed integrally with the cup-shaped extension 259. The flexible arms 203 may extend longitudinally such that they extend up the length of the container 20 when the container 20 is in the storage configuration. The flexible arms 203 may extend up some, a majority or all of the length of the container 20. The flexible arms 203 may be distributed circumferentially around the container 20.

Each of the flexible arms 203 may comprise a projection 204 arranged on an inner face of the flexible arm 203. The projection 204 may be arranged at or near the upper end of the flexible arm 203 such that the projection 204 engages a projection of the container 20 as the container 20 is moved from the storage configuration. The projection of the container 20 may comprise the external protruding ridge 29 extending around the generally cylindrical body 21.Therefore, the flexible arm 203 and/or projection 204 may resist movement of the container 20 towards the outlet of the housing 10.

The flexible arms 203 and/or the projections 204 may flex during movement of the container 20, as shown in Figure 7, such that the container 20 can move past a point at which flexible arms 203 and/or the projections 204 resist movement of the container 20. Therefore, the flexible arms 203 and/or the projections 204 may selectively resist movement of the container 20 towards the outlet of the housing 10. As in Figure 2, Figure 7 shows the device with the container in the storage configuration and the trigger mechanism in the triggered configuration, immediately before the slide 250 begins to move under the action of the biasing element 63.

The flexible arms 203 may be biased towards a centre of the container to thereby create frictional engagement with the container 20 when the container 20 is received in the flexible arms 203 (for example, when container 20 is in the storage configuration). Alternatively, the flexible arms 203 may be in a neutral position when the container 20 is received in the flexible arms 203 and may flex out of their neutral position when the projections 204 interact with the projection of the container 20 to thereby bypass the projection of the container 20.

The arrangement of the flexible arms 203 and/or the position of the projections 204 on the flexible arms and relative to the container 20 may be configured such that the container 20 moves past the point at which the flexible arms 203 and/or the projections 204 provide resistance before the trigger mechanism is moved to the triggered configuration. This configuration may be achieved, for example through the selection of appropriate relative lengths of the flexible arms 203, container 20 and/or the enlarged section 71 of the obstructer 70.

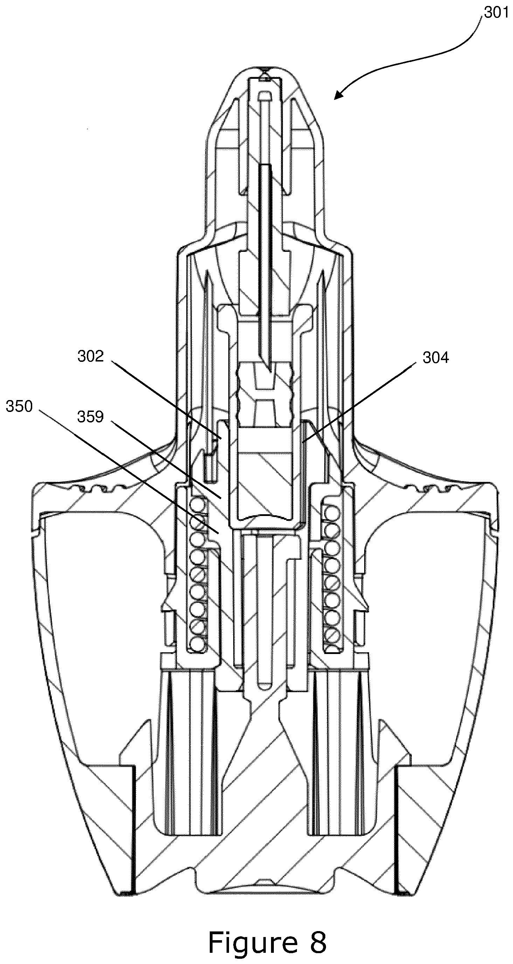

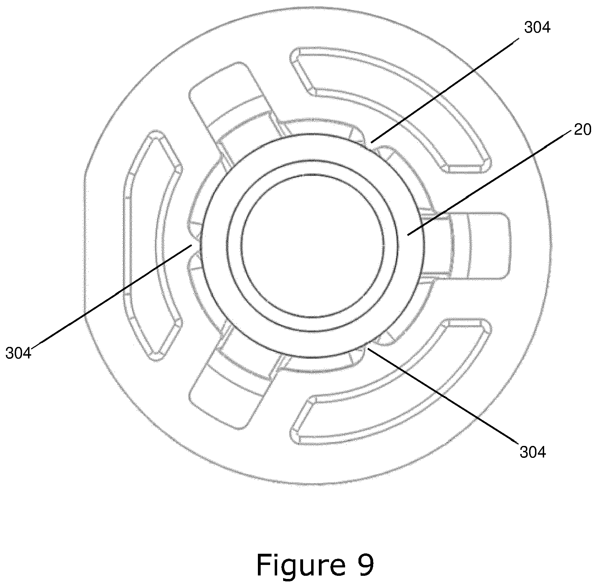

Figures 8 and 9 show another example of a fluid delivery device 301 according to the present disclosure. Only the differences compared to the fluid delivery device 1 previously described will be set out in detail. In all other respects the fluid delivery device 301 may be the same at that already described. Compared to the previous fluid delivery device 1, the fluid delivery device 301 of Figures 8 and 9 has a different configuration of the engagement element 302.

The engagement element 302 may comprise one or more projections 304 in the form of longitudinally extending ribs arranged on the cup-shaped extension 359 of the slide 350, the cup-shaped extension 359 forming a container holder for holding the container 20 when the container is in the storage configuration. The projections 304 may be formed integrally with the cup-shaped extension 359. The projections 304 may be distributed circumferentially around the container 20 and are in contact with an outer face of the container 20 in the storage configuration such that they provide frictional and/or a biased force to the outer face, thereby resisting movement of the container 20. Optionally, the one or more projections 304 may be configured (for example, by selection of an appropriate polymer material) to be plastically deformed by the container 20 on insertion of the container 20 into the cup-shaped extension 359 during assembly of the device. In this way, the projections 304 304 may be deformed by and therefore grip the outer surface of the container 20 as best shown in Figure 9, and may therefore accommodate any variation or tolerance in the diameter of the container 20.

In this example, resistance of the engagement element 302 may be overcome when sufficient force is applied to overcome the friction between the engagement element 302 and the container 20, i.e. when the container 20 is moved sufficiently relative to the engagement element 302 that the engagement element 302 provides reduced or no resistance to further movement of the container 20.

The one or more projections 304 may remain in contact with the container 20 during dispensing to keep the container 20 axially aligned with the container holder throughout dispensing sequence.

The illustrated devices provide examples according to the present disclosure. The skilled person will appreciate that variations of these examples are possible within the scope of the present disclosure. Some possible variations are described below.

The flexible arms may be provided surrounding the container and may be biased radially inwards to grip the container. Alternatively, the flexible arms may be arranged to be received within the container and may be biased radially outwards to grip the container. In other examples, the flexible arms may be arranged in a neutral, unbiased position when the device is in the cocked configuration, and may be deflected by interaction with the container and/or the slide.

As used herein, the term "dispensing configuration" may refer to a relative position of the container in which a delivery channel arranged in or around a piercer or plunger in contact with the container is in fluid communication with the outlet of the housing 10 and the chamber for storage of the fluid in the container. For example, the container may be in the "dispensing configuration" when a piercer has pierced a stopper arranged in an open end of the container and/or a plunger comes into contact with fluid within the container such that fluid from the container is expelled via the outlet of the housing.

As used herein, the term "configured to" may refer to the arrangement, relative sizes and/or shapes and/or relative positions of the components being selected such that the described interaction occurs. For example, the size, shapes and/or relative positions of the engagement element, container and components of the trigger mechanism may be configured such that the engagement element 2 resists movement of the container 20 towards the outlet of the housing 10.

As used herein, the term "selectively resists movement" may refer to the engagement element resisting movement until a certain level of force is applied via the trigger mechanism to move the container to bypass the engagement element and/or to the engagement element resisting movement while the container is in certain configurations, as described herein.

As used herein, the term "overcome the resistance of the engagement element" may refer to the engagement element being physically bypassed or deflected such that the engagement element provides reduced or no resistance to further movement of the container, or that the container has otherwise moved sufficiently relative to the engagement element such that the engagement element provides reduced or no resistance to further movement of the container.

It will be appreciated that the disclosed technology is independent of the exact triggering mechanism, fluid release mechanism or container used in a device, and the disclosed technology may be used with a wide range of triggering mechanisms, fluid release mechanisms and containers as appreciated by those skilled in the art. Exemplary triggering mechanisms include one in which the container may be moved directly by a user applied force rather than by a button that releases a biasing element to axially move the container. Instead of a fluid release mechanism comprising a needle piercing a stopper as shown in the detailed description, a pressure-activated stopper may be used that releases fluid when the pressure inside the container is above a certain threshold. Such a fluid release mechanism is disclosed for example in WO2015008048A1. Examples of containers that may be used with the disclosed technology include standard vials that are filled using vacuum stoppering or tube stoppering. Alternatively, the container may take other forms.

It will also be appreciated that the biasing element may be a spring as shown, or may be any other suitable type of biasing element.

In any example, the piercer may be omitted and an alternative means of facilitating transport of the fluid from the container to the outlet may be provided. For example, the pierceable stopper may be replaced with a piston slidably movable relative to the pump chamber. The device may comprise a piston plunger comprising a delivery channel for delivering fluid discharged from the pump chamber to the outlet of the housing. The piston plunger may be fixedly attached to the housing such that its position relative to the housing is fixed, and may be arranged between the container and the outlet. On movement of the container towards the outlet, the container may move relative to the piston and the piston plunger, causing the fluid to be expelled via an outlet of the piston to the delivery channel and thereby to the outlet. In some examples, the engagement element(s) may be arranged on one or more flexible arms extending from the piston plunger and may engage with the container in the manner described in the first example above. In other examples, the engagement elements may be arranged in any of the other arrangements described above. In any example, the engagement element may be arranged on or be formed as part of the housing.

In the illustrated examples, the trigger mechanism comprises a biasing element. In any example, the trigger mechanism may be configured to move the container in the second movement phase by other means. For example:
the trigger mechanism may be configured to move the container in the second movement phase by continued manual actuation of the slidable button; and/or
the trigger mechanism may comprise a volume of compressed or liquefied gas, the trigger mechanism being configured to move the container in the second movement phase under the action of the compressed or liquefied gas.

In the illustrated examples, the slidable button is shown as a rigid unitary component in which the cup-shaped body and obstructer are integrally formed. In any example, the slidable button may alternatively be formed from multiple parts. For example, the cup-shaped body and obstructer may be formed separately and assembled. The cup-shaped body and obstructer may both be formed from rigid materials which are stationary relative to each other in use. Alternatively, the obstructer may be formed from a rigid component and the cup-shaped body may be flexible such at least part of the cup-shaped body may remain generally stationary relative to the housing in use, and the cup-shaped body may deform in use to accommodate sliding movement of the obstructer on actuation of the slidable button.

In any example, an additional button engagement element may be arranged between the slidable button and the housing. The button engagement element may be arranged such that the resistance of the button engagement element must be overcome by the force of the manual actuation before movement of the slidable button begins.

The terms "upper" and "lower" or similar as used in this application refer to the device when in the orientation shown in the figures, the "upper" end being proximal to the discharge end and the "lower" end being distal to the discharge end of the device in each chase. The terms "outwards" or "inwards" are used to describe directions radially or tangentially away from a central axis of the container and/or the longitudinal axis of the device.

## Claims

1. A fluid delivery device for discharging a fluid comprising:
a) a housing (10) comprising a basal end (10a) and a discharge end (10b) having an outlet for discharging the fluid;
b) a container (20) defining a chamber for storage of the fluid, the container (20) being slideably moveable relative to the housing (10) from a storage configuration to a dispensing configuration;
c) a trigger mechanism configured to apply a force to the container (20) to move the container (20) towards the outlet of the housing (10), the trigger mechanism comprising a manually operated slidable button (30), the slidable button (30) being configured to push the container (20) towards the outlet of the housing (10);
d) an engagement element (2) configured to selectively resist movement of the container (20) towards the outlet of the housing (10); and
e) a biasing element (63);
wherein:
in a first movement phase the trigger mechanism is configured to push the container (20) towards the outlet of the housing (10) to overcome the resistance of the engagement element (2) before the container (20) is moved to the dispensing configuration;
in a second movement phase the trigger mechanism is configured to push the container (20) towards the outlet of the housing (10) such that the container (20) is moved to the dispensing configuration;
in the first movement phase the trigger mechanism is arranged in a cocked configuration in which the trigger mechanism is configured to prevent transfer of a biasing force of the biasing element (63) to the container (20);
the trigger mechanism is configured to move to a triggered configuration during the second movement phase by continued manual actuation of the slidable button (30); and
in the triggered configuration, the trigger mechanism is configured to apply the biasing force to the container (20) to thereby push the container (20) towards the outlet of the housing (10).

2. A fluid delivery device as claimed in any preceding claim wherein the engagement element (2) comprises one or more flexible arms (3).

3. A fluid delivery device as claimed in claim 2 wherein:
the or each of the flexible arms (3) is configured to be inserted into an open end of the container (20) during movement of the container (20) towards the outlet of the housing (10) and is optionally biased outwards to thereby engage with an inner face and/or upper edge of the container (20).

4. A fluid delivery device as claimed in claim 2 wherein the or each of the flexible arms (3) is biased towards a centre of the container (20) to thereby create frictional engagement with the container (20), wherein the or each of the flexible arms (3) is optionally in frictional engagement with an outer face of the container (20).

5. A fluid delivery device as claimed in claim 3 or claim 4 wherein:
the or each of the flexible arms (3) extends along or in the direction of a longitudinal axis of the device; and/or
the or each of the flexible arms (3) extends circumferentially around the container (20) when the container (20) is in the storage configuration.

6. A fluid delivery device as claimed in any one of claims 2 to 5 wherein:
the or each of the flexible arms (3) is arranged between the container (20) and the discharge end (10b) of the housing (10) when the container (20) is in the storage configuration; or
the or each of the flexible arms (3) are arranged laterally to the container (20) and extend along at least part of a length of the container (20) when the container (20) is in the storage configuration.

7. A fluid delivery device as claimed in any one of claims 2 to 6 wherein the or each of the flexible arms (3) extends from an extension of the housing (10).

8. A fluid delivery device as claimed any preceding claim wherein the engagement element (2) comprises one or more protrusions configured to contact a surface of the container (20) to thereby exert a force on the container (20) to selectively resist movement of the container (20) towards the outlet of the housing (10).

9. A fluid delivery device as claimed in claim 8 as dependent on any one of claims 2 to 7, wherein each of the one or more flexible arms (3) comprises one of the one or more protrusions.

10. A fluid delivery device as claimed in claim 8 wherein the or each of the one or more protrusions comprises one or more elongate ribs arranged around a circumference of the container (20).

11. A fluid delivery device as claimed any preceding claim wherein the engagement element (2) is configured to engage the container (20) to thereby selectively resist movement of the container towards the outlet of the housing;
wherein optionally the engagement element (20) is configured to engage one or more of an outer face, an inner face or a rim of an open end of the container (20).

12. A fluid delivery device as claimed in any preceding claim wherein:
the container (20) comprises at least one recess and/or at least one protrusion configured to engage with the engagement element (20); or
the fluid delivery device further comprises a container holder configured to hold the container (20), wherein the engagement element (2) is arranged on the container holder.

13. A fluid delivery device as claimed in any preceding claim wherein the trigger mechanism comprises one or more flexible catch members that in the cocked configuration are catched to the housing (10) and in the triggered configuration are decoupled from the housing (10); and
wherein the slidable button (30) comprises an obstructer (70) that in the cocked configuration engages the one or more flexible catch members to prevent decoupling of the one or more flexible catch members from the housing (10).

14. A fluid delivery device as claimed claim 13, wherein:
the trigger mechanism comprises a slide (50) that is biased by action of the biasing member (63), the slide (63) being arranged to apply the biasing force to the slidable button (30) in the triggered configuration; and
the slide (50) comprises a container holder configured to hold the container (20), wherein the engagement element (2) is arranged on or integrated with the container holder.

15. A fluid delivery device as claimed in any preceding claim further comprising:
a piercer (40) defining a delivery channel for delivering fluid towards the outlet of the housing (10); and
a pierceable stopper (25) arranged within the chamber, the pierceable stopper (25) being configured to be pierced by the piercer (40) so as to discharge the fluid from the chamber along the delivery channel and out of the outlet of the housing (10);
wherein optionally the piercer (40) comprises a needle (41) and a needle holder (42), wherein the engagement element (2) is arranged on the needle holder (42), the needle holder (42) optionally being fixed in position relative to the housing (10).

## Patentansprüche

1. Fluidabgabevorrichtung zum Abführen eines Fluids, umfassend:
a) ein Gehäuse (10), das ein Basisende (10a) und ein Abführende (10b) umfasst, das einen Auslass zum Abführen des Fluids aufweist;
b) einen Behälter (20), der eine Kammer zur Lagerung des Fluids definiert, wobei der Behälter (20) relativ zum Gehäuse (10) von einer Lagerkonfiguration zu einer Ausgabekonfiguration gleitend bewegbar ist;
c) einen Auslösemechanismus, der konfiguriert ist, um eine Kraft auf den Behälter (20) auszuüben, um den Behälter (20) in Richtung des Auslasses des Gehäuses (10) zu bewegen, wobei der Auslösemechanismus einen manuell betätigten Schiebeknopf (30) umfasst, wobei der Schiebeknopf (30) konfiguriert ist, um den Behälter (20) in Richtung des Auslasses des Gehäuses (10) zu drücken;
d) ein Eingriffselement (2), das konfiguriert ist, um einer Bewegung des Behälters (20) in Richtung des Auslasses des Gehäuses (10) selektiv entgegenzuwirken; und
e) ein Vorspannelement (63);
wobei:
in einer ersten Bewegungsphase der Auslösemechanismus konfiguriert ist, um den Behälter (20) in Richtung des Auslasses des Gehäuses (10) zu drücken, um den Widerstand des Eingriffselements (2) zu überwinden, bevor der Behälter (20) in die Ausgabekonfiguration bewegt wird;
in einer zweiten Bewegungsphase der Auslösemechanismus konfiguriert ist, um den Behälter (20) in Richtung des Auslasses des Gehäuses (10) zu drücken, sodass der Behälter (20) in die Ausgabekonfiguration bewegt wird;
in der ersten Bewegungsphase der Auslösemechanismus in einer gespannten Konfiguration angeordnet ist, in der der Auslösemechanismus konfiguriert ist, um Übertragung einer Vorspannkraft des Vorspannelements (63) auf den Behälter (20) zu verhindern;
der Auslösemechanismus konfiguriert ist, um sich während der zweiten Bewegungsphase durch fortgesetzte manuelle Betätigung des Schiebeknopfs (30) in eine ausgelöste Konfiguration zu bewegen; und
in der ausgelösten Konfiguration der Auslösemechanismus konfiguriert ist, um die Vorspannkraft auf den Behälter (20) auszuführen, um dadurch den Behälter (20) in Richtung des Auslasses des Gehäuses (10) zu drücken.

2. Fluidabgabevorrichtung nach einem vorstehenden Anspruch, wobei das Eingriffselement (2) einen oder mehrere flexible Arme (3) umfasst.

3. Fluidabgabevorrichtung nach Anspruch 2, wobei:
der oder jeder der flexiblen Arme (3) konfiguriert ist, um während der Bewegung des Behälters (20) in Richtung des Auslasses des Gehäuses (10) in ein offenes Ende des Behälters (20) eingeführt zu werden und optional nach außen vorgespannt ist, um dadurch mit einer Innenfläche und/oder einer Oberkante des Behälters (20) in Eingriff zu kommen.

4. Fluidabgabevorrichtung nach Anspruch 2, wobei der oder jeder der flexiblen Arme (3) in Richtung einer Mitte des Behälters (20) vorgespannt ist, um dadurch einen Reibungseingriff mit dem Behälter (20) herzustellen, wobei der oder jeder der flexiblen Arme (3) optional in Reibungseingriff mit einer Außenfläche des Behälters (20) steht.

5. Fluidabgabevorrichtung nach Anspruch 3 oder Anspruch 4, wobei:
sich der oder jeder der flexiblen Arme (3) entlang oder in der Richtung einer Längsachse der Vorrichtung erstreckt; und/oder
sich der oder jeder der flexiblen Arme (3) in Umfangsrichtung um den Behälter (20) erstreckt, wenn sich der Behälter (20) in der Lagerkonfiguration befindet.

6. Fluidabgabevorrichtung nach einem der Ansprüche 2 bis 5, wobei:
der oder jeder der flexiblen Arme (3) zwischen dem Behälter (20) und dem Abführende (10b) des Gehäuses (10) angeordnet ist, wenn sich der Behälter (20) in der Lagerkonfiguration befindet; oder
der oder jeder der flexiblen Arme (3) seitlich zum Behälter (20) angeordnet sind und sich über mindestens einen Teil der Länge des Behälters (20) erstrecken, wenn sich der Behälter (20) in der Lagerkonfiguration befindet.

7. Fluidabgabevorrichtung nach einem der Ansprüche 2 bis 6, wobei sich der oder jeder der flexiblen Arme (3) von einer Verlängerung des Gehäuses (10) erstreckt.

8. Fluidabgabevorrichtung nach einem vorstehenden Anspruch, wobei das Eingriffselement (2) einen oder mehrere Vorsprünge umfasst, die konfiguriert sind, um eine Oberfläche des Behälters (20) zu kontaktieren, um dadurch eine Kraft auf den Behälter (20) auszuüben, um einer Bewegung des Behälters (20) in Richtung des Auslasses des Gehäuses (10) selektiv entgegenzuwirken.

9. Fluidabgabevorrichtung nach Anspruch 8, abhängig von einem der Ansprüche 2 bis 7, wobei jeder des einen oder der mehreren flexiblen Arme (3) einen oder mehrere Vorsprünge umfasst.

10. Fluidabgabevorrichtung nach Anspruch 8, wobei der oder jeder des einen oder der mehreren Vorsprünge eine oder mehrere längliche Rippen umfasst, die um den Umfang des Behälters (20) herum angeordnet sind.

11. Fluidabgabevorrichtung nach einem vorstehenden Anspruch, wobei das Eingriffselement (2) konfiguriert ist, um in den Behälter (20) einzugreifen, um dadurch einer Bewegung des Behälters in Richtung des Auslasses des Gehäuses selektiv entgegenzuwirken;
wobei das Eingriffselement (20) optional konfiguriert ist, um in eine oder mehrere von einer Außenfläche, einer Innenfläche oder einem Rand eines offenen Endes des Behälters (20) einzugreifen.

12. Fluidabgabevorrichtung nach einem vorstehenden Anspruch, wobei:
der Behälter (20) mindestens eine Aussparung und/oder mindestens einen Vorsprung umfasst, die konfiguriert sind, um mit dem Eingriffselement (20) in Eingriff zu kommen; oder
die Fluidabgabevorrichtung weiter einen Behälterhalter umfasst, der konfiguriert ist, um den Behälter (20) zu halten, wobei das Eingriffselement (2) auf dem Behälterhalter angeordnet ist.

13. Fluidabgabevorrichtung nach einem vorstehenden Anspruch, wobei der Auslösemechanismus ein oder mehrere flexible Rastelemente umfasst, die in der gespannten Konfiguration am Gehäuse (10) eingerastet sind und in der ausgelösten Konfiguration vom Gehäuse (10) entkoppelt sind; und
wobei der Schiebeknopf (30) einen Blockierer (70) umfasst, der in der gespannten Konfiguration in das eine oder die mehreren flexiblen Rastelemente eingreift, um Entkoppeln des einen oder der mehreren flexiblen Rastelemente vom Gehäuse (10) zu verhindern.

14. Fluidabgabevorrichtung nach Anspruch 13, wobei:
der Auslösemechanismus einen Schieber (50) umfasst, der durch die Wirkung des Vorspannelements (63) vorgespannt ist, wobei der Schieber (63) angeordnet ist, um in der ausgelösten Konfiguration die Vorspannkraft auf den verschiebbaren Knopf (30) auszuüben; und
der Schieber (50) einen Behälterhalter umfasst, der konfiguriert ist, um den Behälter (20) zu halten, wobei das Eingriffselement (2) auf dem Behälterhalter angeordnet oder in diesen integriert ist.

15. Fluidabgabevorrichtung nach einem vorstehenden Anspruch, weiter umfassend:
einen Durchstoßer (40), der einen Abgabekanal zur Abgabe von Fluid zum Auslass des Gehäuses (10) definiert; und
einen durchstoßbaren Stopfen (25), der innerhalb der Kammer angeordnet ist, wobei der durchstoßbare Stopfen (25) konfiguriert ist, um durch den Durchstoßer (40) durchstoßen zu werden, um das Fluid aus der Kammer entlang des Abgabekanals und aus dem Auslass des Gehäuses (10) abzuführen;
wobei der Durchstoßer (40) optional eine Nadel (41) und einen Nadelhalter (42) umfasst, wobei das Eingriffselement (2) auf dem Nadelhalter (42) angeordnet ist, wobei der Nadelhalter (42) optional relativ zum Gehäuse (10) in Position fixiert ist.

## Revendications

1. Dispositif de distribution de fluide pour décharger un fluide comprenant :
a) un boîtier (10) comprenant une extrémité basale (10a) et une extrémité de décharge (10b) présentant une sortie pour décharger le fluide ;
b) un conteneur (20) définissant une chambre de stockage du fluide, le conteneur (20) étant coulissant par rapport au boîtier (10) d'une configuration de stockage à une configuration de distribution ;
c) un mécanisme de déclenchement configuré pour appliquer une force au conteneur (20) afin de déplacer le conteneur (20) vers la sortie du boîtier (10), le mécanisme de déclenchement comprenant un bouton coulissant à commande manuelle (30), le bouton coulissant (30) étant configuré pour pousser le conteneur (20) vers la sortie du boîtier (10) ;
d) un élément d'engagement (2) configuré pour résister sélectivement au mouvement du conteneur (20) vers la sortie du boîtier (10) ; et
e) un élément de rappel (63) ;
dans lequel :
dans une première phase de mouvement, le mécanisme de déclenchement est configuré pour pousser le conteneur (20) vers la sortie du boîtier (10), afin de surmonter la résistance de l'élément d'engagement (2) avant que le conteneur (20) ne soit déplacé vers la configuration de distribution ;
dans une seconde phase de mouvement, le mécanisme de déclenchement est configuré pour pousser le conteneur (20) vers la sortie du boîtier (10), de sorte que le conteneur (20) est déplacé vers la configuration de distribution ;
dans la première phase de mouvement, le mécanisme de déclenchement est agencé dans une configuration armée dans laquelle le mécanisme de déclenchement est configuré pour empêcher le transfert d'une force de rappel de l'élément de rappel (63) au conteneur (20) ;
le mécanisme de déclenchement est configuré pour passer à une configuration déclenchée au cours de la seconde phase de mouvement par actionnement manuel continu du bouton coulissant (30) ; et
dans la configuration déclenchée, le mécanisme de déclenchement est configuré pour appliquer la force de rappel au conteneur (20) pour pousser ainsi le conteneur (20) vers la sortie du boîtier (10).

2. Dispositif de distribution de fluide selon une quelconque revendication précédente, dans lequel l'élément d'engagement (2) comprend un ou plusieurs bras flexibles (3).

3. Dispositif de distribution de fluide selon la revendication 2, dans lequel :
le ou chacun des bras flexibles (3) est configuré pour être inséré dans une extrémité ouverte du conteneur (20) lors du déplacement de ce dernier (20) vers la sortie du boîtier (10), et est poussé en option vers l'extérieur pour ainsi entrer en prise avec une face intérieure et/ou un bord supérieur du conteneur (20).

4. Dispositif de distribution de fluide selon la revendication 2, dans lequel le ou chacun des bras flexibles (3) est poussé vers un centre du conteneur (20) afin de créer un engagement par friction avec le conteneur (20), dans lequel le ou chacun des bras flexibles (3) est, en option, en engagement par friction avec une face extérieure du conteneur (20).

5. Dispositif de distribution de fluide selon la revendication 3 ou la revendication 4, dans lequel :
le ou chacun des bras flexibles (3) s'étend le long, ou dans la direction, d'un axe longitudinal du dispositif ; et/ou
le ou chacun des bras flexibles (3) s'étend circonférentiellement autour du conteneur (20) lorsque celui-ci (20) est en configuration de stockage.

6. Dispositif de distribution de fluide selon l'une quelconque des revendications 2 à 5, dans lequel :
le ou chacun des bras flexibles (3) est agencé entre le conteneur (20) et l'extrémité de décharge (10b) du boîtier (10) lorsque le conteneur (20) est en configuration de stockage ; ou
les ou chacun des bras flexibles (3) sont agencés latéralement au conteneur (20) et s'étendent le long d'au moins une partie de la longueur du conteneur (20) lorsque celui-ci (20) est en configuration de stockage.

7. Dispositif de distribution de fluide selon l'une quelconque des revendications 2 à 6, dans lequel le ou chacun des bras flexibles (3) s'étend depuis une extension du boîtier (10).

8. Dispositif de distribution de fluide selon une quelconque revendication précédente, dans lequel l'élément d'engagement (2) comprend une ou plusieurs saillies configurées pour entrer en contact avec une surface du conteneur (20), afin d'exercer ainsi une force sur le conteneur (20) pour résister sélectivement au mouvement du conteneur (20) vers la sortie du boîtier (10).

9. Dispositif de distribution de fluide selon la revendication 8, lorsque celle-ci dépend de l'une quelconque des revendications 2 à 7, dans lequel chacun parmi le ou les bras flexibles (3) comprend une ou plusieurs saillies.

10. Dispositif de distribution de fluide selon la revendication 8, dans lequel la ou chacune des une ou plusieurs saillies comprend une ou plusieurs nervures allongées agencées autour d'une circonférence du conteneur (20).

11. Dispositif de distribution de fluide selon une quelconque revendication précédente, dans lequel l'élément d'engagement (2) est configuré pour entrer en prise avec le conteneur (20) afin de résister ainsi sélectivement au mouvement du conteneur vers la sortie du boîtier ;
dans lequel l'élément d'engagement (20) est configuré en option pour entrer en prise avec une ou plusieurs d'une face extérieure, d'une face intérieure ou d'un rebord d'une extrémité ouverte du conteneur (20).

12. Dispositif de distribution de fluide selon une quelconque revendication précédente, dans lequel :
le conteneur (20) comprend au moins un évidement et/ou au moins une saillie configurée pour entrer en prise avec l'élément d'engagement (20) ; ou
le dispositif de distribution de fluide comprend en outre un porte-conteneur configuré pour contenir le conteneur (20), dans lequel l'élément d'engagement (2) est agencé sur le porte-conteneur.

13. Dispositif de distribution de fluide selon une quelconque revendication précédente, dans lequel le mécanisme de déclenchement comprend un ou plusieurs éléments d'encliquetage flexibles qui, en configuration armée, sont retenus au boîtier (10) et, en configuration déclenchée, sont désolidarisés du boîtier (10) ; et
dans lequel le bouton coulissant (30) comprend un élément obstructeur (70) qui, dans la configuration armée, entre en prise avec le ou les éléments d'encliquetage flexibles pour empêcher la désolidarisation du ou des éléments d'encliquetage flexibles par rapport au boîtier (10).

14. Dispositif de distribution de fluide selon la revendication 13, dans lequel :
le mécanisme de déclenchement comprend une glissière (50) qui est rappelée par l'action de l'élément de rappel (63), la glissière (63) étant agencée pour appliquer la force de rappel au bouton coulissant (30) dans la configuration déclenchée ; et
la glissière (50) comprend un porte-conteneur configuré pour contenir le conteneur (20), dans lequel l'élément d'engagement (2) est agencé sur ou intégré au porte-conteneur.

15. Dispositif de distribution de fluide selon une quelconque revendication précédente, comprenant en outre :
un perforateur (40) définissant un canal de distribution pour distribuer le fluide vers la sortie du boîtier (10) ; et
un bouchon perforable (25) agencé à l'intérieur de la chambre, le bouchon perforable (25) étant configuré pour être percé par le perforateur (40) afin de décharger le fluide depuis la chambre le long du canal de distribution et par la sortie du boîtier (10) ;
dans lequel en option le perforateur (40) comprend une aiguille (41) et un porte-aiguille (42), dans lequel l'élément d'engagement (2) est agencé sur le porte-aiguille (42), le porte-aiguille (42) étant en option fixé en position par rapport au boîtier (10).
